# EUROPEAN PATENT APPLICATION

(11) **EP 2 374 793 A1**
(43) Date of publication of application: **12.10.2011**
(21) Application number: 09827619.9
(22) Date of filing: 20.11.2009
(51) Int. Cl.: C07C 319/02, C07C 319/26, C07C 321/08, C07B 61/00

(54) **METHOD FOR PRODUCING ALKENYL MERCAPTAN**

(30) Priority: 20.11.2008 JP 2008296436
(71) Applicant: Sumitomo Chemical Company, Limited, Tokyo 104-8260 (JP)
(72) Inventor: SUZUKI, Toshiaki, Niihama-shi Ehime 792-0025 (JP)
(74) Representative: Vossius & Partner
(86) International application number: PCT/JP2009/069702
(87) International publication number: WO 2010/058838

(57) **Abstract**

The present invention relates to a process for producing an alkenyl mercaptan (2), comprising reacting an alkenyl halide (1) with an alkali hydrosulfide, wherein the reaction is carried out in the presence of a disulfide (3) in an amount of 0.5 part by weight or more based on 100 parts by weight of the total amount of the alkenyl halide (1) and the alkenyl mercaptan (2).

## Description

### TECHNICAL FIELD

The present application is filed, claiming the priority based on the Japanese Patent Application No. 2008-296436 (filed on November 20, 2008), and a whole of the contents of the application is incorporated herein by reference.
The present invention relates to a process for producing a compound represented by the formula (2): 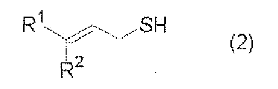 wherein R¹ and R² are independently hydrogen atom or C₁₋₄ alkyl group, [hereinafter sometimes referred to as an alkenyl mercaptan (2)], comprising reacting a compound represented by the formula (1): 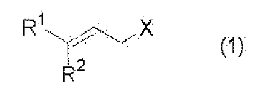 wherein X is chlorine atom, bromine atom or iodine atom and R¹ and R² mean the same as defined above, [hereinafter sometimes referred to as an alkenyl halide (1)], with an alkali hydrosulfide. Alkeny mercaptans (2) are useful, for example, as raw materials for medicines and agricultural chemicals.

### BACKGROUND ART

As a process for producing an alkenyl mercaptan (2) in which an alkenyl halide (1) is reacted with an alkali hydrosulfide, for example, JP 2007-204453 A discloses a process in which an allyl chloride is used as the alkenyl halide (1) and is reacted with a sodium hydrosulfide to produce an allyl mercaptan as the alkenyl mercaptan (2).

### DISCLOSURE OF THE INVENTION

### PROBLEM TO BE SOLVED BY THE INVENTION

However, the above-described process may generate a large amount of compounds represented by the formula (4): 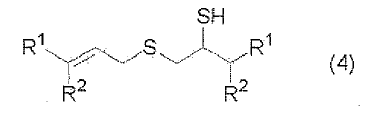 wherein R¹ and R² mean the same as defined above, [hereinafter sometimes referred to as a byproduct (4)], besides the intended alkenyl mercaptan (2), and thus, the alkenyl mercaptan (2) prepared by this process may not necessarily have satisfactory quality.

An object of the present invention is therefore to provide a process for producing a high-quality alkenyl mercaptan (2) while the generation of a byproduct (4) is sufficiently suppressed.

The present inventor has found that the generation of a byproduct (4) can be suppressed by carrying out the reaction in the presence of a predetermined disulfide compound in an amount of at least a predetermined amount. In addition, he has found that the disulfide compound is capable of stabilizing the alkenyl mercaptan (2).

### MEANS FOR SOLVING THE PROBLEM

That is, the present invention provides a process for producing a compound represented by the formula (2): 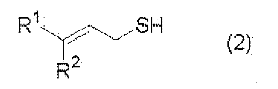 wherein R¹ and R² are independently hydrogen atom or
C₁₋₄ alkyl group,
comprising reacting a compound represented by the formula (1) : 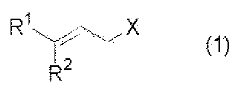 wherein X is chlorine atom, bromine atom or iodine
atom and R¹ and R² mean the same as defined above, with an alkali hydrosulfide, wherein the above reaction is carried out in the presence of a compound represented by the formula (3) : 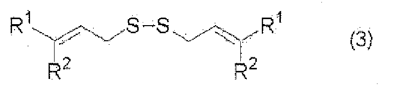 wherein R¹ and R² mean the same as defined above, [hereinafter sometimes referred to as a disulfide (3)], in an amount of 0.5 part by weight or more based on 100 parts by weight of the total amount of the compounds represented by the formulae (1) and (2).

The present invention also provides a process for stabilizing an alkenyl mercaptan (2), in which 0.5 to 6.0 parts by weight of a disulfide (3) is added to the alkenyl mercaptan (2) based on 100 parts by weight of the alkenyl mercaptan (2).

### EFFECT OF THE INVENTION

According to the present invention, a high-quality alkenyl mercaptan (2) can be produced while sufficiently suppressing the generation of a byproduct (4), and additionally, the alkenyl mercaptan (2) can be sufficiently stabilized.

### MODES FOR CARRYING OUT THE INVENTION

Hereinafter, the present invention will be described in detail.
In the present invention, a compound represented by the formula (1) (or an alkenyl halide (1)): 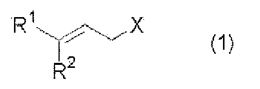 wherein R¹ and R² is independently hydrogen atom or C₁₋₄ alkyl group, and X is chlorine atom, bromine atom or iodine atom,
is reacted with an alkali hydrosulfide.

In the formula (1), examples of the C₁₋₄ alkyl group include methyl group, ethyl group, n-propyl group, isopropyl group, n-butyl group, sec-butyl group and tert-butyl group. Examples of the alkenyl halide (1) include 2-propenyl halides (allyl halides) such as 2-propenyl chloride (allyl chloride), 2-propenyl bromide (allyl bromide) and 2-propenyl iodide (allyl iodide); 2-butenyl chloride (crotyl chloride); 3-methyl-2-butenyl chloride; 2-pentenyl chloride; 2-hexenyl chloride; 2-heptenyl chloride and the like. Of those, when the 2-propenyl halides (allyl halides) are used as raw materials, the process of the present invention is effectively employed.

Examples of the alkali hydrosulfide include lithium hydrosulfide, sodium hydrosulfide, potassium hydrosulfide and the like, among them, sodium hydrosulfide is preferable. The alkali hydrosulfide may contain an alkali sulfide, an alkali carbonate, an alkali sulfite or alkali thiosulfate. Such an alkali hydrosulfide may be used in the form of flakes or in the form of an aqueous solution obtained by dissolving them in water.

Particularly from the viewpoint of a quality of the alkenyl mercaptan as will be described later, preferably, 2.0 to 10.0 parts by weight of an alkali sulfide is added to the reaction system, based on 100 parts by weight of an alkali hydrosulfide. As examples of a method for adding a predetermined amount of the alkali sulfide to the reaction system, there may be mentioned a method of adding an alkali hydrosulfide containing a predetermined amount of an alkali sulfide to the reaction system; or a method of adding a predetermined amount of an alkali sulfide to the reaction system together with an alkali hydrosulfide.

In the present invention, the reaction is carried out in the presence of a compound represented by the formula (3) (or disulfide (3)): 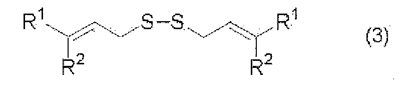 wherein R¹ and R² mean the same as defined above, in an amount of 0.5 part by weight or more based on 100 parts by weight of the total amount of the alkenyl halide (1) and the alkenyl mercaptan (2).

This leads to a result that a high-quality alkenyl mercaptan (2) can be produced while sufficiently suppressing the generation of a compound represented by the formula (4) (or byproduct (4)): 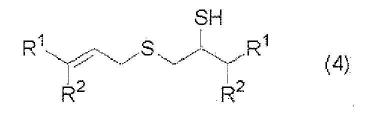 wherein R¹ and R² mean the same as defined above.

The lower limit of the amount of the disulfide (3) to be present is 0.5 part by weight, preferably 5.0 parts by weight, more preferably 5.5 parts by weight, based on 100 parts by weight of the total amount of the alkenyl halide (1) and the alkenyl mercaptan (2). The upper limit of the amount of the disulfide (3) is not limited, but it is preferably 6.0 parts by weight or less based on 100 parts by weight in total amount of the alkenyl halide (1) and the alkenyl mercaptan (2), in consideration of a load in the subsequent purification.

The disulfide (3) is a reaction byproduct which can be generated derived from an alkali sulfide and an alkenyl mercaptan (2), when an alkali hydrosulfide containing an alkali sulfide is used in the reaction. As a method for making at least predetermined amount of the disulfide (3) present in the reaction system, there may be mentioned a method in which a predetermined amount of an alkali sulfide is added to the reaction system; or a method in which the disulfide (3) is directly added to the reaction system.

When the reaction is carried out with the alkali sulfide in the reaction system, the amount of the alkali sulfide to be added to the reaction system is preferably 2.0 parts by weight or more, more preferably 8.0 parts by weight or more, particularly 9.0 parts by weight or more, and preferably 10.0 parts by weight or less, more preferably 9.8 parts by weight or less, particularly 9.5 parts by weight or less, based on 100 parts by weight of the alkali hydrosulfide, so that the above-described amount of the disulfide (3) is present in the reaction system.

When the disulfide (3) is directly added to the reaction system, examples of the disulfide (3) include diallyl disulfide, allyl propyl disulfide and the like. The amount of the disulfide (3) to be added is preferably from 1 to 4 parts by weight, more preferably from 1.5 to 2.5 parts by weight, based on 100 parts by weight of the alkenyl halide (1).

The amount of the alkali hydrosulfide to be used is usually one mole or more, preferably from 1.05 to 2 moles per mole of the alkenyl halide (1).

The above-described reaction is usually carried out in a solvent. As the solvent, an organic solvent may be singly used, but it is preferable to use an oil-water two-phase solvent mixture composed of an organic solvent and water. Examples of the organic solvent include aliphatic hydrocarbons such as hexane, heptane and octane; alicyclic hydrocarbons such as cyclopentane and cyclohexane; aromatic hydrocarbons such as benzene, toluene and xylene; halogenated aliphatic hydrocarbons such as dichloromethane, chloroform and 1,2-dichloroethane; ethers such as diethyl ether and dibutyl ether; esters such as ethyl acetate and butyl acetate; and the like. Among them, the aromatic hydrocarbons are preferably used.

The amount of the solvent to be used is usually from about 0.5 to about 10 times larger than the amount of the alkenyl halide (1) in terms of weight. When a solvent mixture of an organic solvent and water is used, the amount of the organic solvent to be used is usually from about 0.5 to about 5 times lager than the amount of the alkenyl halide (1) in terms of weight; and the amount of water to be used is usually from about 0.5 to about 5 times larger than the amount of the alkali hydrosulfide in terms of weight. A ratio of the organic solvent to the water in terms of a weight ratio of the organic solvent/the water is usually from about 1/5 to about 5/1.

When the oil-water two-phase solvent mixture composed of an organic solvent and water is used in the reaction, the reaction is carried out preferably in the presence of a phase transfer catalyst. Examples of the phase transfer catalyst include quaternary ammonium salts such as tetra-n-ethylammonium bromine, tetra-n-ethylammonium chloride, tetra-n-butyammonium bromide, tetra-n-butylammonium chloride, tetra-n-butylammonium hydrogensulfate and triethylbenzylammonium chloride; quaternary phosphonium salts such as tetra-n-butylphosphonium bromide; crown ethers; cryptands; and the like. Among them, the quaternary ammonium salts are preferably used. The amount of the phase transfer catalyst to be used is usually 0.001 to 0.2 times larger, preferably 0.05 to 0.1 times larger, in terms of mole, than the amount of the alkenyl halide (1).

The reaction method may be appropriately selected. However, preferably, the reaction is carried out by supplying the alkenyl halide (1) to a mixture of the alkali hydrosulfide and the solvent. On the other hand, when the disulfide (3) is directly added to the reaction system, the disulfide (3) may be added to any of the solvent, the alkenyl halide (1) and the alkali hydrosulfide. When the phase transfer catalyst is used, the phase transfer catalyst may be added to any of the solvent, the alkenyl halide (1) and the alkali hydrosulfide.

When the alkenyl halide (1) is supplied, it is preferable to supply a cooled alkenyl halide (1) from the viewpoint of suppression of generation of impurities. A cooling temperature is usually from about -20 to 50°C, although it may vary depending on the kind of the alkenyl halide (1) to be used.

A temperature for the reaction of the alkenyl halide (1) with the alkali hydrosulfide is usually from 0 to 100°C, preferably from 30 to 50°C. The reaction is usually carried out under around a normal pressure, or optionally under an increased pressure or a reduced pressure.

Thus, a reaction mixture containing the alkenyl mercaptan (2) can be obtained. As an operation for a posttreatment after the reaction, a known method such as washing or distillation may be employed.

The disulfide (3) is a compound which improves storage stability of the alkenyl mercaptan (2). The alkenyl mercaptan (2) can be stably stored by adding the disulfide (3) in an amount of 0.5 part by weight or more, preferably 5.0 parts by weight or more, more preferably 5.4 parts by weight or more, and 6.0 parts by or less, preferably 5.8 parts by weight or less, more preferably 5.6 parts by weight or less, based on 100 parts by weight of the alkenyl mercaptan (2). In this regard, the alkenyl mercaptan (2) is usually stored under a nitrogen gas atmosphere. A storage temperature is from 0°C to about a room temperature. The alkenyl mercaptan (2) may be diluted with the organic solvent mentioned above before the storage thereof.

### EXAMPLES

Hereinafter, Examples of the present invention will be illustrated, which however should not be construed as limiting the scope of the present invention in any way. In Examples, a yield of an allyl mercaptan [i.e., a compound of the formula (2) in which each R¹ and R² is hydrogen atom] and the respective contents of a diallyl disulfide [i.e., a compound of the formula (3) in which each R¹ and R² is hydrogen atom] and a bydproduct (4) [herein, a compound of the formula (4) in which each R¹ and R² is hydrogen atom] were analyzed by gas chromatography and were then calculated.
Conditions for analysis:

| | | |
|---|---|---|
| Column | | DB-WAX 1.0 µm, 0.53 mmφ X 30 m |
| Column temperature | : | 40°C (10 mins.) → 5°C/min. → 230°C (5 mins.) |
| Carrier gas | : | He 5 ml/min. |
| Injection temperature | : | 150°C |
| Detection temperature | : | 235°C |
| Detector | : | FID |

### Example 1

A glass reactor equipped with a reflux condenser, a thermometer, a stirrer and a dropping funnel with a jacket was charged with sodium hydrosulfide hydrate (184.91 g, 1.484 mol)(a sodium hydrosulfide content = 45% by weight, a sodium sulfide content = 2.40% by weight, and sodium sulfide/sodium hydrosulfide = 5.3% by weight), water (36.43 g), xylene (82.63 g) and an aqueous solution (57.26 g, 0.175 mol) of 69.6% by weight of triethylbenzylammonium chloride, and the resulting mixture was stirred. A nitrogen gas was introduced into a gas-phase layer in the reactor to form a nitrogen gas stream.
Then, allyl chloride (103.05 g, 1.319 mol) was charged in the dropping funnel with the jacket and was cooled to a temperature of from -2 to 5°C. The cooled allyl chloride was added dropwise over 7 hours while maintaining a temperature of the reaction solution at 40°C, and then the resulting mixture was further maintained at 40°C for 3 hours. The content of the diallyl disulfide in the organic phase determined at the start of the heat retention (i.e. zero (0) hour of the heat retention time) was 2.46% by weight. The obtained reaction solution was cooled to a temperature of from 0 to 10°C and was then admixed with water (123.60 g) so as to dissolve the precipitated sodium chloride. Then, the solution was subjected to oil-water separation to obtain 174.17 g of a solution of allyl mercaptan in xylene as an organic phase. The content of the diallyl disulfide relative to the allyl chloride and the allyl mercaptan in the solution was 2.62% by weight, and the content of the byproduct (4) was 0.32% by weight. The yield of the allyl mercaptan relative to the allyl chloride was 83.90%.

### Example 2

A glass reactor equipped with a reflux condenser, a thermometer, a stirrer and a dropping funnel with a jacket was charged with sodium hydrosulfide hydrate (179.12 g, 1.437 mol) (a sodium hydrosulfide content = 45% by weight, a sodium sulfide content = 0.50% by weight, and sodium sulfide/sodium hydrosulfide = 1.1% by wight), water (35.30 g), xylene (80.00 g), an aqueous solution (55.60 g, 0.170 mol) of 69.6% by weight of triethylbenzylammonium chloride and diallyl disulfide (3.95 g, 0.025 mol), and the resulting mixture was stirred. A nitrogen gas was introduced into a gas-phase layer in the reactor to form a nitrogen gas stream. In this regard, the addition amount of diallyl disulfide is 3.9% by weight based on an addition amount of allyl chloride described later.
Then, allyl chloride (100.11 g, 1.282 mol) was charged in the dropping funnel with the jacket and was cooled to a temperature of from -2 to 5°C. The cooled allyl chloride was added dropwise over 7 hours while maintaining a temperature of the reaction solution at 40°C, and then the resulting mixture was further maintained at 40°C for 3 hours. The content of the diallyl disulfide in the organic phase determined at the start of the heat retention (i.e. zero (0) hour of the heat retention time) was 3.52% by weight. The obtained reaction solution was cooled to a temperature of from 0 to 0°C and was then admixed with water (120.02 g) so as to dissolve the precipitated sodium chloride. Then, the solution was subjected to oil-water separation to obtain 177.72 g of a solution of allyl mercaptan in xylene as an organic phase. The content of the diallyl disulfide relative to the allyl chloride and the allyl mercaptan in the solution was 3.72% by weight, and the content of the byproduct (4) was 0.02% by weight. The yield of the allyl mercaptan relative to the allyl chloride was 94.19%.

### Example 3

A glass reactor equipped with a reflux condenser, a thermometer, a stirrer and a dropping funnel with a jacket was charged with sodium hydrosulfide hydrate (179.06 g, 1.437 mol) (a sodium hydrosulfide content = 45% by weight, a sodium sulfide content = 0.60% by weight, and sodium sulfide/sodium hydrosulfide = 1.3% by weight), water (35.25 g), xylene (80.00 g), an aqueous solution (55.60 g, 0.170 mol) of 69.6% by weight of triethylbenzylammonium chloride and sodium sulfide pentahydrate (13.80 g, 0.080 mol), and the resulting mixture was stirred. A nitrogen gas was introduced into a gas-phase layer in the reactor to form a nitrogen gas stream. In this regard, the total addition amount of sodium sulfide is 9.3% by weight, based on the addition amount of sodium hydrosulfide.
Then, allyl chloride (100.03 g, 1.281 mol) was charged in the dropping funnel with the jacket and was cooled to a temperature of from -2 to 5°C. The cooled allyl chloride was added dropwise over 7 hours while maintaining a temperature of the reaction solution at 40°C, and then the resulting mixture was further maintained at 40°C for 3 hours. The content of the diallyl disulfide in the organic phase determined at the start of the heat retention (i.e. zero (0) hour of the heat retention time) was 1.04% by weight. The obtained reaction solution was cooled to a temperature of from 0 to 10°C and was then admixed with water (120.08 g) so as to dissolve the precipitated sodium chloride. Then, the solution was subjected to oil-water separation to obtain 169.09 g of a solution of allyl mercaptan in xylene as an organic phase. The content of the diallyl disulfide relative to the allyl chloride and the allyl mercaptan in the solution was 1.12% by weight, and the content of the byproduct (4) was 0.23% by weight. The yield of the allyl mercaptan relative to the allyl chloride was 75.36%.

### Comparative Example 1

A glass reactor equipped with a reflux condenser, a thermometer, a stirrer and a dropping funnel with a jacket was charged with sodium hydrosulfide hydrate (179.11 g, 1.438 mol) (a sodium hydrosulfide content = 45% by weight, a sodium sulfide content = 0.60% by weight, and sodium sulfide/sodium hydrosulfide = 1.3% by weight), water (35.78 g), xylene (80.05 g) and an aqueous solution (55.62 g, 0.170 mol) of 69.6% by weight of triethylbenzylammonium chloride, and the resulting mixture was stirred. A nitrogen gas was introduced into a gas-phase layer in the reactor to form a nitrogen gas stream.
Then, allyl chloride (100.62 g, 1.319 mol) was charged in the dropping funnel with the jacket and was cooled to a temperature of from -2 to 5°C. The cooled allyl chloride was added dropwise over 7 hours while maintaining a temperature of the reaction solution at 40°C, and the resulting mixture was further maintained at 40°C for 4 hours. The content of the diallyl disulfide in the organic phase determined at the start of the heat retention (i.e. zero (0) hour of the heat retention time) was 0.41% by weight. The obtained reaction solution was cooled to a temperature of from 0 to 10°C and was then admixed with water (120.00 g) so as to dissolve the precipitated sodium chloride. Then, the solution was subjected to oil-water separation to obtain 168.63 g of a solution of allyl mercaptan in xylene as an organic phase. The content of the diallyl disulfide relative to the allyl chloride and the allyl mercaptan in the solution was 0.43% by weight, and the content of the byproduct (4) was 2.53% by weight. The yield of the allyl mercaptan relative to the allyl chloride was 90.66%.

### Example 4

A solution of allyl mercaptan in xylene, comprising 5.70 parts by weight of diallyl disulfide and 0.03 part by weight of the byproduct (4) based on 100 parts by weight of allyl mercaptan, was stored at 5°C under a nitrogen gas atmosphere for 48 hours. The content of the byproduct (4) determined after 48 hours had passed was 0.08 part by weight per 100 parts by weight of the allyl mercaptan. The content of the byproduct (4) was increased only 0.05 part by weight compared with the content of that determined at the start of the storage, and thus, the solution showed the favorable storage stability.

## Claims

1. A process for producing a compound represented by the formula (2): 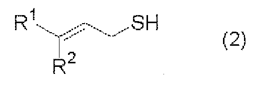 wherein R¹ and R² are independently hydrogen atom or a
C₁₋₄ alkyl group,
comprising reacting a compound represented by the formula (1) : 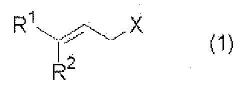 wherein X is chlorine atom, bromine atom or iodine
atom and R¹ and R² mean the same as defined above, with an alkali hydrosulfide, wherein said reaction is carried out in the presence of a compound represented by the formula (3): 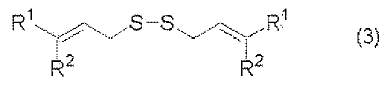 wherein R¹ and R² mean the same as defined above,
in an amount of 0.5 part by weight or more based on 100 parts by weight of the total amount of the compounds represented by the formulae (1) and (2).

2. The process of Claim 1, wherein said reaction is carried out in the presence of a phase transfer catalyst in a two-phase solvent composed of an organic solvent and water.

3. The process of Claim 1 or 2, wherein said reaction is carried out in a reaction system in which 2.0 to 10.0 parts by weight of an alkali sulfide is added based on 100 parts by weight of an alkali hydrosulfide.

4. A process for stabilizing a compound represented by the formula (2): 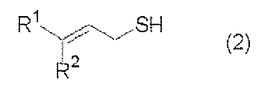 wherein R¹ and R² are independently hydrogen atom or
C₁₋₄ alkyl group,
wherein 0.5 to 6.0 parts by weight of a compound represented by the formula (3): 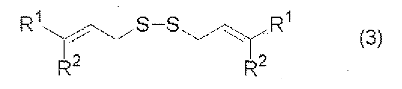 wherein R¹ and R² mean the same as defined above,
is added to the compound represented by the formula (2) based on 100 parts by weight of the compound represented by the formula (2).
